Europäisches Patentamt

European Patent Office

Office européen des brevets

(11) Veröffentlichungsnummer: **0 152 905**
**B1**

(12) **EUROPÄISCHE PATENTSCHRIFT**

(45) Veröffentlichungstag der Patentschrift: **30.01.91**

(21) Anmeldenummer: **85101526.3**

(22) Anmeldetag: **13.02.85**

(51) Int. Cl.⁵: **G 01 S 5/18**

(54) **Verfahren und Vorrichtung zum Messen des Ortes mehrerer Messpunkte mit Hilfe von Ultraschallimpulsen.**

(30) Priorität: **21.02.84 DE 3406212**
**21.02.84 DE 3406210**
**21.02.84 DE 3406179**
**21.02.84 DE 3406180**

(43) Veröffentlichungstag der Anmeldung:
**28.08.85 Patentblatt 85/35**

(45) Bekanntmachung des Hinweises auf die
Patenterteilung:
**30.01.91 Patentblatt 91/05**

(84) Benannte Vertragsstaaten:
**AT BE CH DE FR GB IT LI LU NL SE**

(56) Entgegenhaltungen:
**EP-A-0 075 273**
**DE-A-3 025 234**
**FR-A-2 305 742**
**FR-A-2 372 435**
**GB-A-2 004 369**
**US-A-3 821 469**

(73) Patentinhaber: **Travenol GmbH**
**Nymphenburger Strasse 1**
**D-8000 München 2 (DE)**

(72) Erfinder: **Schumpe, G. Dr. Dr.**
**Tannenweg 53**
**D-2509 Meckenheim/Merl (DE)**
Erfinder: **Hansen, Markus, Dr.**
**Goethestrasse 46**
**D-5300 Bonn 1 (DE)**
Erfinder: **Hofmann, Peter, Prof. Dr.**
**Weberstrasse 105**
**D-5300 Bonn 1 (DE)**

(74) Vertreter: **Gudel, Diether, Dr. et al**
**Patentanwälte Dr. V. Schmied-Kowarzik Dipl.-**
**Ing. G. Dannenberg Dr. P. Weinhold Dr. D. Gudel**
**Dipl.-Ing. S. Schubert Dr. P. Barz Grosse**
**Eschenheimer Strasse 39**
**D-6000 Frankfurt am Main 1 (DE)**

EP 0 152 905 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

(56) Entgegenhaltungen:

**IEEE TRANSACTIONS ON INSTRUMENTATION AND MEASUREMENT, Band IM-26, Nr. 1, März 1977, Seiten 5-10, New York, US; W.E. MORITZ et al.: "A system for locating points, lines, and planes in space"**
**Aufsatz "Ganguntersuchungen und funktionelle Wirbelsäulenvermessungen mittels eines neu entwickelten Echtzeit-Stereo-Ultraschall-Topometers (ESUT)" von G. Schumpe et al., in Buch "Funktionelle Diagnostik in der Orthopädie", Ferdinand Enke Verlag, Stuttgart 1979, Seiten 69-72**

**Beschreibung**

Die Erfindung betrifft ein Verfahren für den Einsatz in der medizinischen Diagnose, in der medizinischen Rehabilitation und im Sportunterricht, zum Messen des Orts mehrerer Meßpunkte in einem vorgegebenen Koordinatensystem mit Hilfe von Ultraschallimpulsen, wobei man an jedem Meßpunkt einen Ultraschallsender befestigt sowie wenigstens drei raumfeste Ultraschallempfänger vorsieht und man die Ultraschallsender nacheinander zu Stoßwellen erregt, deren jeweilige Laufzeit zu den Ultraschallempfängern man mißt und daraus mit Hilfe einer datenverarbeitenden Anlage den Ort der Meßpunkte bestimmt, wobei man durch ein oftmaliges Wiederholen der Messung in kurzen Absbänden eine gegebenenfalls vorhandene Bewegung der Meßpunkte mißt sowie gegebenenfalls daraus abgeleitete Größen.

Außerdem bezieht sich die Erfindung auf eine Schaltungsanordnung zur Durchführung dieses Verfahrens, wobei die Schaltungsanordnung an Meßpunkten befestigte Ultraschallsender, raumfeste Ultraschallempfänger und eine mit den Sendern und Empfängern verbundene datenverarbeitende Anlage aufweist, sowie auf Ultraschallsender einer solchen Schaltungsanordnung.

Ein solches Verfahren und eine derartige Schaltungsanordnung sind beschrieben in einem Aufsatz "Ganguntersuchung und funktionelle Wirbelsäulenvermessungen mittels eines neu entwickelten Echtzeit-Stereo-Ultraschall-Topometers (ESUT)" von G. Schumpe, G. H. Hansen, G. Syndicus und H. Rössler, von denen die ersten beiden Erfinder dieses Patentes, erschienen in dem Buch "Funktionelle Diagnostik in der Orthopädie", Ferdinand Enke Verlag, Stuttgart 1979, Seiten 69—72. Diese sogenannte Echtzeit-Ultraschall Topometer wird dort für die medizinsche Diagnose eingesetzt, insbesondere zur Darstellung von Bewegungsabläufen von Patienten an zu untersuchenden Körperstellen, beispielsweise am Kniegelenk oder an der Wirbelsäule. Dort verwendet man Ultraschallsender, die bei ihrem Anstoß eine kugelsymmetrische Stoßwelle abstrahlen.

Dieses bekannte Verfahren hat sich an und für sich bewährt. Die vorliegende Erfindung bezweckt eine weitere Ausgestaltung dieses bekannten Verfahrens bzw. dieser bekannten Vorrichtung.

Bei dem bekannten Verfahren hat man Stoßwellen verwendet, die nach ihrer Definition erst nach einer Vielzahl von Schwingungen auf die Amplitude Null abfielen. Auch ist dort nichts über die Steilheit der Anstiegsflanke dieser Stoßwellen ausgesagt. Die dort verwendeten verhältnismäßig langen Wellenzüge für die Stoßwellen führen aber zu einem verhältnismäßig schlechten Auflösungsvermögen, weil man este das Abklingen des Wellenzuges abwarten muß, ehe man den nächsten Ultraschallimpuls aussenden kann. Schnellere Bewegungen lassen sich nach diesem bekannten Verfahren also nicht befriedigend darstellen bzw. auflösen.

Hingewiesen sei auch noch auf die deutsche Offenlegungsschrift DE—A—24 22 837, die ein Verfahren und eine Vorrichtung zum Messen dreidimensionaler Koordinaten von Modellen beschreibt, und zwar ebenfalls mit Hilfe von Ultraschallimpulsen. Hierzu wird ein Sender an einem Koordinatensystem befestigt und die Laufzeiten der ausgesendeten Ultraschallimpulse werden gemessen und ergeben nach ihrer Auswertung das auszumessende Profil in den drei Koordinaten. Ebenfalls wird dort ein Taster beschrieben, der aus einem länglichen Stift besteht, an dem voneinander beabstandet zwei Ultraschallsender befestigt sind, die an einer Seite des Stiftes angebracht sind. In einer Verlängerung der diese beiden Ultraschallsender verbindenden Linie befindet sich eine gegenüber dem eigentlichen Taststift abgebogene Tastspitze dieses Tasters. Dieser Taster wird dann von Hand über die abzutastende Fläche geführt, wobei wiederum die Laufzeiten der Ultraschallimpulse zu einem raumfesten Empfänger ausgemessen und ausgewertet werden. Hieran ist es aber insbesondere nachteilig, daß man beim Abtasten der Kontur der Fläche stets sorgfältig darauf achten muß, daß die beiden Sender ihre Impulse gerichtet zum Empfänger abstrahlen, well der Empfänger sonst die Signale nicht mehr mit ausreichender Stärke empfängt. Auch muß man bei Abtasten darauf achten, daß der Taststift möglichst genau parallel zu sich bleibt. Kippt man nämlich den Taster um einen wesentlichen Winkel, so kann ebenfalls der Empfänger aus dem Abstrahlungsbereich der Sender geraten und zusätzlich wird hierbei das durch die beiden Sender und den Empfänger gebildete Meßdreieck so verzerrt, daß diese Verzerrung anschließend in der angeschlossenen datenverarbeitenden Anlage nicht mehr korrigiert werden kann. Diese Einschränkungen sind für einen handgeführten Taster in der Praxis unzuträglich. Diese Druckschrift enthält keinen Hinweis über die Art der abzustrahlenden Ultraschallwellen. Es ist dort noch nicht einmal angegeben, daß man Ultraschallimpulse verwenden soll.

Hingewiesen sei auch auf einen Aufsatz der Erfinder der vorliegenden Anmeldung, erschienen in der Zeitschrift für Orthopädie, Band 120, Heft 2, April 1982, Seiten 115 bis 119. In diesem Aufsatz werden medizinische Anwendungen für das Verfahren der Echtzeit-Ultraschall-Topometrie gegeben, nämlich bei der operativen Behandlung des Kniegelenks. In diesem Zusammenhang sie erwähnt, daß eine oftmalige, schnell aufeinanderfolgende Wiederholung der vorstehend beschriebenen Messung des Ortes eines Meßpunktes in dem Koorinatensystem die Bewegung dieses Meßpunktes in dem Koordinatensystem ergibt. Wegen der praktisch nicht vorhandenen Zeitverzögerung zwischen der Ausführung der Bewegung und deren Messung, ggfs, einschließlich deren Sichtbarmachung, beispielsweise auf einem Monitor, erhält die beobachtende Person, ggfs. der behandelnde Arzt oder auch der Patient selbst, sofort eine Darstellung der durchgeführten Bewegung. Aus diesem Grunde wird von einer Echtzeit-Topometrie gesprochen. Auch von diesem Prinzip macht die vorliegende Erfindung Gebrauch.

Zum Stand der Technik wird auch verwiesen auf die FR—A—2 372 435. Diese Druckschrift beschreibt ein Verfahren und eine Vorrichtung zum Orten eines unter Wasser befindlichen Gegenstandes mit Hilfe von Schallwellen. Hier sind mehr als drei Schallsender vorgesehen, die zwischen sich einen Raum aufspannen. Auch ist eine herkömmliche Schaltungsanordnung zum Ansteuern der Ultraschallsender und für die Verarbeitung der von den Empfängern ankommenden Signale beschrieben.

Die EP—A1—00 75 273 beschreibt einen Ultraschallsender, der zwei gleichformatige, übereinander gelegte und miteinander verklebte Plättchen aus piezokeramischen Material aufweist. Mit den Plättchen ist ein Schalltrichter verbunden. Vor dem Schalltrichter befindet sich eine Lochblende.

Fernerhin sei darauf hingewiesen, daß im vorstehenden und im nachfolgenden von Ultraschallsendern und Ultraschallempfängern gesprochen wird, wobei in der Regel angegeben wird, daß sich der Sender am Meßpunkt befindet und die Empfänger ortsfest, d.h. unbeweglich sind. Es können aber die Sender und die Empfänger auch gegeneinander vertauscht werden, weil es nur auf die Laufzeiten der zwischen Sender und Empfänger ausgestrahlen Impulse ankommt. In diesem Fall ist dann der Empfänger am Meßpunkt und die Sender sind ortsfest. Weiterhin sei in diesem Zusammenhang erwähnt, daß man wenigstens vier dieser Geräte braucht, d.h. wenigstens drei Sender und einen Empfänger oder wenigstens drei Empfänger und einen Sender. Auch dies beruht auf einfachen geometrischen Überlegungen.

Ausgehend von einem eingangs genannten Verfahren, wie es in dem erwähnten Aufsatz, erschienen in dem Buch "Funktionelle Diagnostik in der Orthopädie", 1979, Seiten 69—72, beschrieben ist, liegt daher der Erfindung die Aufgabe zugrunde, dieses Verfahren so zu verbessern, daß insbesondere die Auflösung bei der Messung des Ortes des Meßpunktes weiterhin fühlbar verbessert wird. Auch sollen Messungen bei sich schnell bewegenden Meßpunkten möglich sein.

Zur Lösung dieser Aufgabe ist das erfindungsgemäße Verfahren dadurch gekennzeichnet, daß die Ultraschallsender in einer vorgegebenen Reihenfolge nacheinander zu Stoßimpulsen erregt werden, wobei diese Reihenfolge während der Messung gleich bleibt, und daß die zeitliche Verzögerung zwischen den Stoßimpulsen jeder Meßreihe als vorgegebene Verzögerungszeit so groß gewählt wird, daß, auch bei sich relativ zueinander bewegenden Ultraschallsendern, deren Ultraschallimpulse stets in der gleichen Reihenfolge von den Ultraschallempfängern empfangen werden, wobei die vorgegebene Verzögerungszeit in einem Vorversuch bestimmt wird und wenigstens gleich der größten bei der Messung zu erwartenden Verzögerungszeit der bewegten Sender untereinander gewählt wird, und wobei die Anstiegsflanke des Stoßimpulses möglichst steil ist und weniger als 90° beträgt und der Stoßimpuls höchstens sechs Schwingungen enthält.

Man verwendet erfindungsgemäß also Stoßimpulse, die sehr rasch gedämpft werden. Vorzugsweise ist der Stoßimpuls schon nach zwei, drei oder maximal vier vollen Schwingungen vollständig gedämpft. Die Anstiegsflanke des Stoßimpulses soll aber möglichst steil sein, damit die volle Energie des Impulses sofort zur Verfügung steht. Allerdings soll die Anstiegsflanke unter 90° bleiben, well sonst die Schwingungsenergie vollständig in der den Sender umgebenden Luft absorbiert wird. Die Vorteile des erfindungsgemäßen Verfahrens bestehen darin, daß praktisch keine Analysezeit benötigt wird, um die Frequenz zu erkennen. Es ergeben sich sehr kurze Totzeiten, die sonst dadurch bedingt werden, daß die nächste Messung erste dann durchgeführt werden kann, wenn der Impuls wieder zur Ruhe gekommen ist. Damit verbunden ergibt sich also beim erfindungsgemäßen Verfahren eine besonders hohe räumliche und zeitliche Auflösung. In erster Linie denkt man beim erfindungsgemäßen Verfahren daher an eine oftmalige Wiederholung der Messung des Maßpunktes zwecks Darstellung seiner Bewegung in den Raumkoordinaten, üblicherweise in kartesischen Koordinaten. Es kann aber auch ein anderes Koordinatensystem verwendet werden, beispielsweise Polarkoordinaten. Die Anstiegsflanke des erfindungsgemäß verwendeten Stoßimpulses soll so gewählt werden, daß seine Absorption in der Umgebungsluft, die mit wachsender Steilheit der Anstiegsflanke wächst, und die an die Umgebungsluft abgegebenen Schallintensität, die ebenfalls mit wachsender Steilheit der Anstiegsflanke wächst, in einem vernünftigen Verhältnis zueinander stehen. Üblicherweise wird man Anstiegsflanken zwischen 45° und 80° verwenden, und zwar eher im oberen Bereich.

Bei vielen Anwendungsfällen wird man mehrere der Ultraschallsender an entsprechenden Meßpunkten befestigen, insbesondere bei medizinischen Anwendungen. Damit die nachgeschaltete datenverarbeitende Anlage erkennen kann, welches der Empfangssignale der Empfänger welchem Sender zugeordnet ist, werden die Ultraschallsender zeitlich nacheinander zu den Stoßimpulsen erregt.

Es gibt nun Anwendungsfälle, bei denen sich die Entfernung zwischen den mehreren Sendern und dem oder den Empfängern während einer Meßreihe ständig ändert, beispielsweise wenn ein Patient auf einem Laufband läuft. Hierbei kann es also vorkommen, daß, bedingt durch diese Abstandsänderungen, die Impulse beim Empfänger in einer Reihenfolge ankommen, die anders ist als die Reihenfolge bei der vorhergehenden Messung. Um auch hier die richtige Zuordnung der Impulse in ihrer zeitlichen Abfolge sicherzustellen, sind die weiteren zitierten Maßnahmen vorgesehen.

Hierzu bestimmt man in einem kurzen Vorversuch die größte Verzögerungszeit der Sender untereinander, die während beispielsweise des Laufens des Patienten auf dem Laufband zu erwarten ist. Man stellt dann an der Elektronik den zeitlichen Abstand in der nacheinander erfolgenden Zündung der Sender so ein, daß während dieser Meßreihe die Impulse an den Empfängern stets in dieser vorgegebenen Reihenfolge eintreffen, und zwar unabhängig davon, ob einige der Sender während der Messung andere der Sender räumlich überholen oder nicht. Diese zeitlichen Verzögerungen wird man aus praktischen Gründen einander gleich wählen, damit man mit einer einzigen Einstellung auskommt. Diese Maßnahmen ergeben eine fühlbar erhöhte Rechnergeschwindigkeit, weil der Rechner jetzt weiß, daß bei allen Versuchsbedingungen die Impulse stets in der vorgegebenen Reihenfolge beim Empfänger ankommen.

Man verwendet Ultraschallsender, die zu Stoßimpulsen erregt werden können, deren Anstiegsflanken möglichst steil sind und die weniger als 90° betragen, wobei der Stoßimpuls höchstens sechs Schwingungen enthält.

Es wird bevorzugt, wenn der Ultraschallsender an einem Ende eines Taststiftes befestigt ist, dessen anderes Ende als Tastspitze ausgebildet ist. Man erhält dadurch einen gewissermaßen glatt durchgehenden Taststift, der problemlos sowohl von Hand wie auch über eine geeignete Führungsbuchse oder dergleichen geführt werden kann, ohne daß hierbei die Ausbreitung der Ultraschallwellen gestört wird.

Eine wichtige Ausgestaltung der Erfindung ist in Anspruch 2 gekennzeichnet, wobei man die Empfänger, nicht wie bei dem geschilderten Vorbild nur in einer Ebene vor der betreffenden Person anordnet, sondern an beliebigen Orten außerhalb dieser Ebene. Hier ist zu berücksichtigen, daß die Sender die Impulse bevorzugt in einer Richtung nach vorne abstrahlen. Bewegt sich nun die Person, so konnte es beim Stand der Technik passieren, daß der Kegel der Schallabstrahlung aus dem Empfangsbereich der Empfänger fiel, bzw. daß die Impulse durch Körperteile der Person absorbiert wurden, so daß diese Information verlorenging. Diese nachteile werden durch die Maßnahmen nach Anspruch 2 vermieden. Auch kann man hierdurch Reflexionen der Impulse an den Wänden des Raums besser ausschalten.

Nach Anspruch 3 wird durch den außerhalb der Ebene angeordneten vierten Empfänger eine zweite Ebene aufgespannt, die zu der ersten Ebene senkrecht steht. Allgemein wird man alle Empfänger auf der Oberfläche eines Quaders anordnen, was zu einer fühlbaren Vereinfachung der Auswertung der Meßsignale führt. Die Empfänger ordnet man beispielsweise an den Eckpunkten eines Quaders an, wobei die Abstände zwischen den Empfängern ausreichend groß sein sollen, um eine gut definierte Basis für die Meßstrecken zu bieten. Ggfs. wird man die Empfänger auch hinter oder seitlich der betreffenden Person bzw. der Meßpunkte anordnen, um auch in diese Richtungen abgestrahlte Signale empfangen zu können.

Patentanspruch 5 gibt wichtige Merkmale an, mit denen erreicht wird, daß die Meßwerte mit großer Genauigkeit und Schnelligkeit zur Verfügung stehen und angezeigt werden.

Sobald der Zeitgeber einen Impuls abgibt, wird der betreffende Ultraschallsender zu einem Stoßimpuls erregt, der sich durch eine hohe Amplitude und damit durch einen hohen Energieinhalt sowie durch eine nur kurzzeitige Erregung mit sofort daran anschließenden Abfall auf Null auszeichnet. Der Stoßimpuls hat vorzugsweise nur eine einzige Anstigsflanke und geht dann sofort auf die Intensität Null zurück, oder doch im wesentlichen auf Null. Fehlmessungen werden dadurch weitestgehend ausgeschlossen. Gleichzeitig mit der Aussendung des Startimpulses über den Zeitgeber wird der Zähler auf Null gesetzt, der jetzt die ihm fortlaufend zugeführten Schwingungen des HF-Oszillators zählt. Diese Zahl wird in den empfängsseitigen Speicher übertragen, sobald dieser den Empfangsimpuls erhält. Die im Speicher befindliche Zahl ist somit ein direktes Maß für das Zeitintervall zwischen dem Aussenden des Stoßimpulses und dessen Empfang im Empfänger. Aufgrund der bekannten Fortpflanzungsgeschwindigkeit des Schalls in dem betreffenden Medium, im allgemeinen in Luft, kann daraus direkt die Entfernung zwischen Sender und Empfänger angezeigt werden.

Es ist auch möglich, die Schaltungsanordnung so zu treffen, daß der Zähler die ihm vom HF-Oszillator zugeleiteten Impulse ständig an den empfängerseitigen Speicher weiterleitet, der dann die betreffende Zahl anzeigt, sobald er den zugehörigen Empfangsimpuls erhält.

Auf die beschriebene Art und Weise werden alle Impulse zwischen einem Sender und wenigstens drei Empfängern verarbeitet, und zwar unter Zuhilfenahme der erwähnten EDV-Anlage einschließlich ihres Programms.

Mit der beschriebenen Vorrichtung ist es ohne weiteres möglich, den Ort des Meßpunktes innerhalb einer Kugel mit einem Durchmesser kleiner als 1 mm zu bestimmen.

Diese Messungen werden kurz nacheinander wiederholt, wobei die Wiederholungsfrequenz durch die Frequenz des Zeitgebers bestimmt ist. Dies ist daher vorzugsweise einstellbar, beispielsweise zwischen Werten von 1 und 100 Hz. Je nach der jeweils verwendeten Meßanordnung, wobei insbesondere die Zahl der jeweils verwendeten Sender und Empfänger eine Rolle spielt sowie das zur Auswertung der Signale eingesetzte Software-Programm und natürlich auch die Schmelligkeit des verwendeten Rechners, wird man die Frequenz des Zeitgebers auf einen passenden Wert einstellen, der natürlich eine den jeweiligen Umständen angepaßte gute zeitliche Auflösung ergeben sollte. Versuche haben gezeigt, daß bei Frequenzen um 40 Hz bei einer nicht zu großen Anzahl von Sendern und Empfängern gute Meßergebnisse erzielbar sind.

Die Schwingungsfrequenz des HF-Oszillators stellt man vorteilhaft so ein, daß die in der Anzeige erscheinende Zahl, die, wie erläutert, proportional zur Laufzeit des Stoßimpulses ist und damit auch proportional zur Entfernung zwischen Sender und Empfänger, direkt eine Anzeige für die jeweils gemessene Entfernung in dem betreffenden Maßsystem, im allgemeinen im metrischen System, gibt. Nachdem die Schallgeschwindigkeit in Luft bei einer Temperatur von 20°C bekanntlich 343,8 m/sek. beträgt, bringt es daher für den Schaltungsaufwand und die Anzeige Vorteile, wenn man die Frequenz des HF-Oszillators auf einen Zahlenwert einstellt, der diesem Zahlenwert entspricht, multipliziert mit $10^n$, wobei n eine Zahl=1, 2, 3... ist. Vorzugsweise ist n=4, wobei dann die auf der Anzeige erscheinenden Zahlen die jeweils gemessene Entfernung direkt in ein Zehntel Millimeter wiedergeben. Dieselben Überlegungen gelten natürlich auch für Anzeigen in anderen Maßsystemen, beispielsweise im Zoll-System.

Nachdem die Schallgeschwindigkeit in Luft temperaturabhängig ist, kann auch die Frequenz des HF-Oszillators einstellbar sein, um zur Erzielung einer noch verbesserten Meßgenauigkeit auch Temperaturschwankungen oder Abweichungen von der erwähnten Zimmertemperatur von 20°C zu berücksichtigen.

Es wurde bereits erwähnt, daß es wesentliche Vorteile mit sich bringt, wenn der Ultraschallsender Stoßimpulse abgibt. Um dies zu erreichen, gibt es mehrere konstruktive Möglichkeiten. Besondere Vorteile gibt ein Ultraschallsender aus piezokeramischem Material, obgleich auch andere Prinzipien verwendet werden können, wie weiter unten noch näher erläutert werden wird.

Ein solcher Ultraschallsender ist vorzugsweise dadurch gekennzeichnet, daß er zwei gleichformatige, übereinander gelegte und miteinander verklebte Plättchen aus piezokeramischen Material aufweist, die so polarisiert sind, daß sie bei Anlegen einer Spannung vorgegebener Polarität an ihre metallisierten Außenflächen kugelschalenförmig in der einen Richtung und bei Spannungsumkehr in der anderen Richtung ausgelenkt werden und die an ihrer Unterseite im Bereich ihrer Kanten schwingungsgedämpft an einem Gehäuse befestigt sind. Dieser Ultraschallsender strahlt eine nahezu ideale Kugelwelle nullter bzw. erster Ordnung aus, verbunden mit einer hohen, stoßartig ansteigenden Amplitude, d.h. mit anderen Worten den angestrebten Stoßimpuls. Auch dies wird weiter unten noch näher erläutert.

Bevorzugt wird es, wenn der Durchmesser der Plättchen klein ist im Vergleich zu der Wellenlänge des abzustrahlenden Schalls in dem piezokeramischen Material. Bei diesen Bedingungen wird ein Kugelstrahler nullter Ordnung erreicht.

Versuche haben gezeigt, daß sich besonders gute Ergebnisse erzielen lassen, wenn die Plättchen quadratisch sind und nur an ihren Ecken am Gehäuse befestigt sind.

Bei vielen Anwendungsfällen ist es vorteilhaft, wenn die Ultraschallsender die Stoßimpulse in einem möglichst großen Raumwinkel abstrahlen, um nämlich sicherzustellen, daß jeder Stoßimpuls von wenigstens drei Empfängern empfangen und ausgewertet wird, und zwar unabhängig von den jeweiligen Bewegungen und auch Verschwenkungen des Meßpunktes mitsamt dem an ihm befestigten Ultraschallsender. Andererseits hat die Abstrahlung in einem großen Raumwinkel den Nachteil einer fühlbar verringerten Empfangsleistung. Hierzwischen wird man also einen Kompromiß suchen.

Um zu erreichen, daß der Stoßimpuls in einem möglichst großen Winkel im Raum abgestrahlt wird, wird es bevorzugt, wenn eine Lochblende dicht vor der schallabstrahlenden Fläche der Plättchen angeordnet ist. An der Kante der Lochblende wird der Stoßimpuls gebeugt und erreicht somit die gewünschte Raumabstrahlung. Der Durchmesser des Lochs der Lochblende soll in der Größenordnung der Wellenlänge liegen, um zu große Energieverluste durch die Blende zu verhindern. Gute Ergebnisse wurden mit Lochblenden mit einem Durchmesser von etwa 4 mm bei einer Schallwellenlänge von 7 mm erzielt.

Die Lochblende wird vorzugsweise sehr nahe, beispielsweise in einem Abstand von etwa 0,1 mm, vor der schwingenden Fläche der Plättchen angebracht. Dadurch wird der akustische Kurzschluß, den die Rückseite der Membran (=schwingende Plättchenanordnung) mit ihrer Vorderseite hat, zum größten Teil blockiert und die Energieabstrahlung weiterhin verbessert. Es dient demselben Zweck, wenn auf die schallabstrahlende Fläche der Plättchen ein Schalltrichter aufgesetzt ist.

Die Erfindung wird im folgenden anhand von Ausführungsbeispielen näher erläutert, aus denen sich weitere wichtige Merkmale ergeben. Es zeigt:

Fig. 1 schematisch den abgegebenen Stoßimpuls, wobei dessen Amplitude über der Zeit aufgetragen ist;

Fig. 2 ein Blockschaltbild zur Erläuterung der zur Anwendung kommenden elektrischen Schaltung;

Fig. 3 perspektivisch und schematisch eine erfindungsgemäße Vorrichtung zur Ausmessung von Meßpunkten an einem Menschen, wobei die Empfänger im Raum angeordnet sind;

Fig. 4 schematisch und perspektivisch einen erfindungsgemäßen Ultraschallsender zur Erläuterung des grundsätzlichen Aufbaus dieses Senders;

Fig. 5 in einer Seitenansicht den Ultraschallsender nach Fig. 4 im Ruhezustand;

Fig. 6 den Sender nach Fig. 4 und 5 in vorgespanntem Zustand;

Fig. 7 den Sender nach Fig. 4 und 6 bei der Abgabe eines Ultraschall-Stoßimpulses.

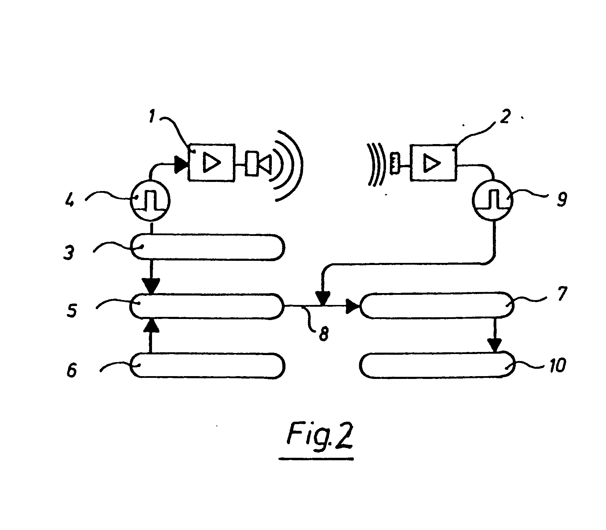

Zunächst sie das Meßverfahren in seinen Grundzügen anhand von Fig. 2 erläutert, die eine nur eindimensionale Meßstrecke zeigt mit einem einzigen Ultraschallsender 1 und einem einzigen Ultraschallempfänger 2.

Ein frei schwingender Oszillator 3, der beispielsweise auf eine Frequenz von 25 Hz eingestellt ist, gibt an einen Senderverstärker 4 beispielsweise alle 40 msec einen Startimpuls, worauf der Sender 1 dann ein Ultraschallsignal abgibt. Gleichzeitig wird ein Zähler 5 auf Null gesetzt, der daraufhin die Impulse eines Quarzoszillators 6 zu zählen beginnt. Der Oszillator 6 schwingt beim angenommenen Beispiel mit 3,438 MHz.

Nach einer bestimmten Laufzeit des vom Sender 1 abgegebenen Ultraschallimpulses, welche Laufzeit von der Entfernung zwischen Sender und Empfänger abhängt, wird der Impuls im Empfänger 2 empfangen. In diesem Augenblick sendet der Empfänger 2 ein Übernahmesignal an einen Zwischenspeicher 7 (Latsch-Zwischenspeicher), der daraufhin den gegenwärtigen Zählerstand des Zählers 5 (über die Leitung 8) übernimmt. Das Empfangssignal ist vorher in einem Empfängerverstärker 9 verstärkt worden. Der vom Zähler 5 in den Zwischenspeicher 7 übernommene Zählerstand wird im Speicher 7 bis zum entsprechenden Impuls bei der nächsten Einzelmessung gespeichert. Ein Treiber (Decoder-Treiber) stellt den Inhalt des Speichers 7 jeweils auf einer Anzeige 10 dar. Dies ist beispielsweise ein Sieben-Segment-Leuchtdioden-Display.

Da die Schallgeschwindigkeit in Luft (bei einer Temperatur von 20°C) genau 343,8 m pro Sekunde beträgt, zeigt die Anzeige 10 die Entfernung zwischen Sender 1 und Empfänger 2 in ein Zehntel mm an. Die Temperaturabhängigkeit beträgt dabei 0,2% pro 1° Temperaturdifferenz, gemessen in Grad Celsius. Mit anderen Worten wird die Anzeige durch die Abstimmung der Schwingungen des Oszillators 6 auf die Schallgeschwindigkeit in Luft vereinfacht. Der Oszillator 6 kann in seiner Schwingungsfrequenz auch so geregelt werden, daß er die erwähnte Temperaturabhängigkeit der Schallgeschwindigkeit in Luft selbsttätig berücksichtigt. Natürlich kann er auch auf andere Frequenzen eingestellt werden, wobei dann die Anzeige 10 entsprechend geeicht werden muß.

Auf die beschriebene Weise erhält man somit in der Anzeige 10 die Entfernung zwischen Sender 1 und Empfänger 2, und zwar bei diesem Beispiel auf Zehntel Millimeter genau. Widerholt man die vorstehend beschriebene Entfernungsmessung bei sich bewegendem Sender 1 und/oder Empfänger 2, so gibt die Anzeige 10 anschließend die neue Frequenz wider. Weil der Oszillator 3 als Zeitgeber wirkt und beispielsweise mit einer Frequenz von 25 Hz schwingt, werden die Messungen beim angegebenen Beispiel 25 mal pro Sekunde wiederholt. Dies ergibt eine sehr gute Darstellung bei nicht zu schnellen Bewegungen zwischen Sender 1 und Empfänger 2.

Die Anzeige 10 kann beispielsweise auch als Analogschreiber, Fernsehschirm-Monitor und/oder Drucker ausgebildet sein. Weil im Speicher 7 alle Daten zur Verfügung stehen, können daraus auch andere Größen abgeleitet werden, beispielsweise die Geschwindigkeit, mit der sich der Abstand zwischen Sender 1 und Empfänger 2 ändert, die daraus abgeleitete Beschleunigung usw. Alles dies kann ebenfalls zur Anzeige gebracht werden. Der Baustein 7 ist dann kein reiner Speicher mehr, sondern wird zu einer elektronischen Datenverarbeitungsanlage (EDV-Anlage) ausgebaut.

Verwendet man nun nicht nur einen Empfänger 2, sondern drei räumlich voneinander getrennte Empfänger, die raumfest angeordnet sind, und gibt man in die EDV-Anlage deren Abstände relativ zu einem vorgegebenen Koordinatensystem ein, so kann man auch die Bewegung des Senders 1 im Raum messen. Bei diesem Beispiel wird angenommen, daß der Sender 1 bewegt wird und die Empfänger 2 raumfest angeordnet sind. Es ist aber auch eine umgekehrte Anordnung mit raumfesten Sender 1 und beweglichen Empfängern 2 grundsätzlich möglich, wenngleich von der Software der EDV-Anlage her aufwendiger.

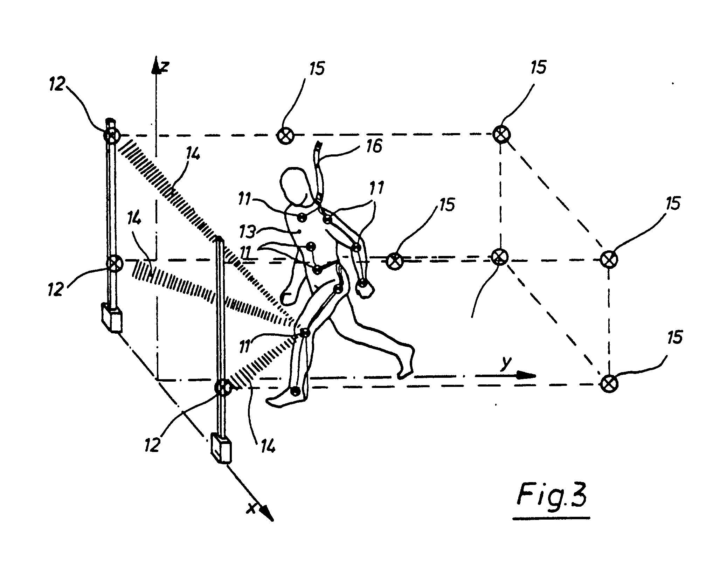

Fig. 3 zeigt eine Anwendung zu diagnostischen oder therapeutischen Zwecken, bei der am Körper einer zu untersuchenden Person 13 mehrere Ultraschallsender 11 befestigt sind, deren Stoßimpulse von mehreren raumfesten Ultraschallempfängern 12 aufgefangen werden. Die Ultraschallsender 11 sind an den jeweils interessierenden Körperpartien befestigt, wie dies weiter unten noch näher erläutert wird. Die Sender 11 strahlen Ultraschall-Stoßwellen mit möglichst kugelförmiger Charakteristik und einem gegebenen Öffnungswinkel ab, den man auf die Reichweite der betreffenden Meßanordnung abstimmt. Raumfest sind mehrere der Ultraschallempfänger 12 vorgesehen, die so angeordnet sind, daß jede der bei Pos. 14 angedeuteten Ultraschallwellen von wenigstens dreien der Empfänger 12 aufgenommen wird. Als Beispiel ist gezeigt, daß in einer Ebene von der Untersuchungsperson 13 insgesamt drei der Empfänger 12 angeordnet sind, und zwar an den Ecken eines gedachten Quaders.

Weitere Ultraschallempfänger 15 sind zusätzlich vorgesehen, und zwar außerhalb der durch die Ultraschallempfänger 12 definierten Ebene. Diese zusätzlichen Ultraschallempfänger 15 können an den Ecken, Kanten oder Flächen des zeichnerisch angedeuteten, gedachten Quaders angeordnet sein, aber auch sonst an beliebigen Stellen im Raum. Sie sollen jeweils so angeordnet werden, daß sie, abhängig von der jeweils durchgeführten Messung, sicher stellen, daß zumindest drei der Ultraschallempfänger 12, 15 jeden Ultraschallimpuls aufnahmen. Dies ermöglicht eine eindeutige Bestimmung des Ortes des Meßpunktes. Man kennt nämlich die Abstände der drei Empfänger voneinander, außerdem—über die Laufzeiten der Impulse—die Abstände jedes Senders 11 von jedem der Empfänger 12, 15. Durch diese Daten ist eine Pyramide definiert, deren Spitze der Sender 11 ist. Bevorzugt wird es, wenn die Empfänger 12, 15 an Stativen, Stangen oder anderen geeigneten reflexionsarmen Halterungen im Raum einstellbar angebracht werden können.

Die Stromversorgung für die Ultraschallsender 11 ist bei Pos. 16 angedeutet.

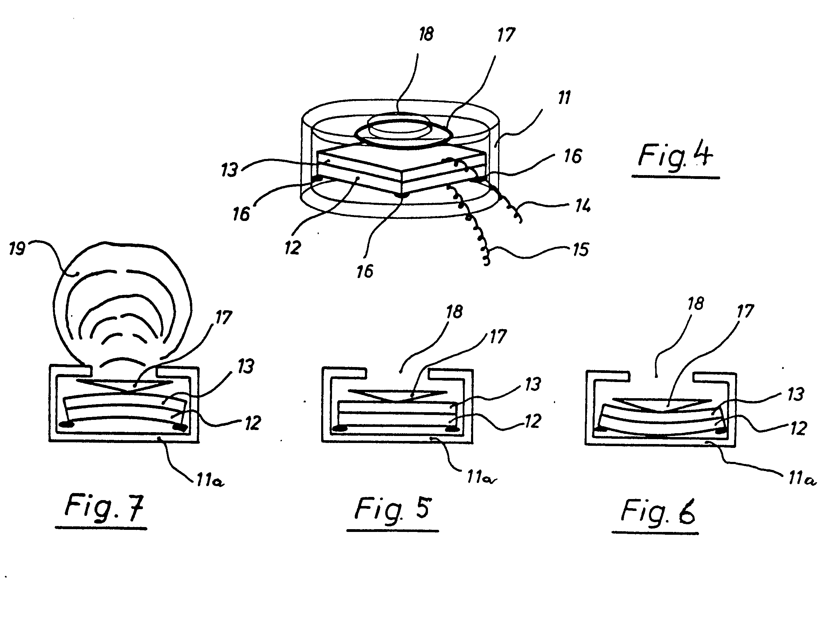

Der in den Fig. 4 bis 7 gezeigte Ultraschallsender besteht in seinen wesentlichen Bauteilen aus einem Gehäuse 11a, in dem eine schwingende Membran untergebracht ist. Diese besteht aus zwei gleichformatigen und übereinander gelegten sowie entgegengesetzt polarisierten piezokeramischen Plättchen 12, 13, die miteinander verklebt sind. Über einen Anschlußleiter 14 kann eine elektrisches Potential auf die metallisierte Oberfläche des oberen Plättchens 13 aufgebracht werden und über einen weiteren Anschlußleiter 15 ein elektrisches Potential an die metallisierte Unterseite des unteren Plättchens 12 angelegt werden.

Beim gezeigten Ausführungsbeispiel sind die beiden Plättchen 12, 13 quadratisch mit Kantenlängen von etwa 8 mm. Die aus den Plättchen 12, 13 bestehende schwingende Membran ist an ihren vier Ecken über Körper 16 aus Silikonkautschuk am Gehäuse 11 befestigt. Auf die Mitte der Oberseite der Membran ist weiterhin ein schallabstrahlender Trichter 17, vorzugsweise aus Kunststoffmaterial, aufgesetzt und dort mit der Membran verklebt.

Das Gehäuse 11 umschließt die Membran mit ihren Bauteilen allseitig. Nur an der Oberseite, das ist die schallabstrahlende Seite, ist über ein Loch 18 eine Blende im Gehäuse angebracht.

Die Figuren 5 bis 7 erläutern die Funktionsweise dieses Ultraschallsenders. Figur 5 zeigt den Ruhezustand, bei dem als keine Spannung über die Anschlußleiter 14, 15 an der Membran anliegt. In Figur 6 ist die Membran nach unten, d.h. zum Boden des Gehäuses 11 hin, vorgespannt. Dies wird durch die Anlage einer entsprechenden Spannung an die Membran erzielt.

Soll jetzt ein Stoßimpuls abgegeben werden, so wird die Spannung umgepolt und über den vorstehend beschriebenen piezokeramischen Effekt, der an sich bekannt ist, nimmt die Membran die in Figur 7 gezeigte Gestalt ein und der Stoßimpuls wird über die Lochblende mit dem Loch 18 abgegeben, wie bei Pos. 19 angedeutet. Daraus ist auch ersichtlich, daß eine gute Raumcharakteristik erzielt wird.

Die Aufhängung der Membran an den Kanten bzw. Ecken bringt den Vorteil einer erhöhten Amplitude mit sich, weil, wie ein Vergleich der Figuren 6 und 7 besonders deutlich ergibt, dann der mittlere Bereich der Membran aus der vorgespannten Lage nach Figur 6 über die Ruhelage nach Figur 5 in die Abstrahlungslage nach Figur 6 geht, und zwar innerhalb kürzester Zeit. Die Aufhängung der Membran über die kleinen Körper aus Silikonkautschuk oder einem anderen, schalldämmenden Material, dämpft die Schwingung sehr gut, so daß sie tatsächlich einen Impulscharakter bekommt. Der Schalltrichter 17 verstärkt die Schallabstrahlung, und die Lochblende mit dem Loch 18 ist so bemessen, daß sie einerseits über die Beugung am Rand des Lochs 18 den Abstrahlungswinkel des Stoßimpulses im Raum vergrößert und andererseits aber noch ausreichend Schallenergie durch das Loch 18 hindurchtreten kann.

Grundsätzlich können auch andere Ultraschallsender bei der erfindungsgemäßen Vorrichtung verwendet werden. Eine gute Kugelcharakteristik hat beispielsweise eine aus einem kleinen Luftvolumen bestehende schwingende Kugel, die durch plötzliches Erhitzen expandiert. Eine solche schwingende Kugel hat den Vorteil, daß eine optimale Anpassung der Impedanzen zwischen Schallstrahler und Schalleiter gegeben ist, weil beides Luft ist. Im Prinzip wird ein solcher Schallstrahler aufgebaut wie ein Ionenhochtöner eines modernen Lautsprechers, bei dem eine Metallspitze mit einem extrem kleinen Krümmungsradius amplitudenmodulierte Hochfrequenz aussendet, die durch den starken Feldstärkegradienten Luftmoleküle ionisiert und durch die Amplitudenmodulation in Schwingungen versetzt. Der Wirkungsgrad eines solchen Wandlersystems ist bis zirka 100 KHz sehr gut, so daß es grundsätzlich für den erfindungsgemäßen Zweck geeignet ist. Das große und schwere Gehäuse und die relativ starre Hochfrequenzzuleitung lassen aber die Verwendung eines solchen Schallstrahlers für die Messung feiner Bewegungsstrukturen, die auch durch das Meßgerät möglichst wenig beeinflußt werden sollen, als nachteilig erscheinen.

Ein solcher Luftkugelschallstrahler, der nur eine Druckwelle aussenden soll, läßt sich grundsätzlich auch durch einen kurzzeitig gezündeten Lichtbogen erzeugen. Solche Lichtbogensender arbeiten aber nicht sehr zuverlässig und müssen außerdem mit einer Spannung in der Größenordnung von mehreren Kilovolt betrieben werden, was wiederum sicherheitstechnische Probleme mit sich bringt, insbesondere bei der Anwendung der erfindungsgemäßen Vorrichtung an Patienten, Sportlern und anderen Personen. Auch wäre ein solcher Sender relativ unhandlich und schwer, während der beschriebene Ultraschallsender nach Figuren 4 bis 6 sich durch sehr kleine Abmessungen und ein geringes Gewicht auszeichnet sowie durch verhältnismäßig niedrige, ihm zuzuführende Spannungen, die keine besonderen Sicherheitsvorkehrungen notwendig machen.

Versuche haben ergeben, daß das zeitliche Auflösungsvermögen der beschriebenen Vorrichtung bei der Verwendung von sechs Ultraschallsendern etwa gleich ein Dreißigstel Sekunde ist. Dieser Wert wird bei Verwendung von weniger Sendern noch verbessert.

Verglichen mit anderen, bisher bekannten Meßsystemen, die vorzugsweise optisch über Filmaufnahmen arbeiten, zeichnet sich die erfindungsgemäße Vorrichtung durch eine etwa dreißigfach bis einhundertfach verbesserte Ortsauflösung auf. Je nach der verwendeten Anzahl der Sender und Empfänger und auch der Rechnergeschwindigkeit ergibt sich eine Meß- und Analysenzeit von wenigen Minuten bis herab zu etwa ein Hunderstel Sekunde. Eine solch kurze Zeitspanne zwischen dem Ereignis (Bewegung) und dessen Darstellung erschließt neue Anwendungsgebiete, beispielsweise für die medizinische Rehabilitation und für den Sportunterricht, wobei dann die betreffende Person selbst, gegebenenfalls zusammen mit einem Arzt oder Sportlehrer, die betreffenden Bewegungen praktisch momentan sieht und kontrollieren kann.

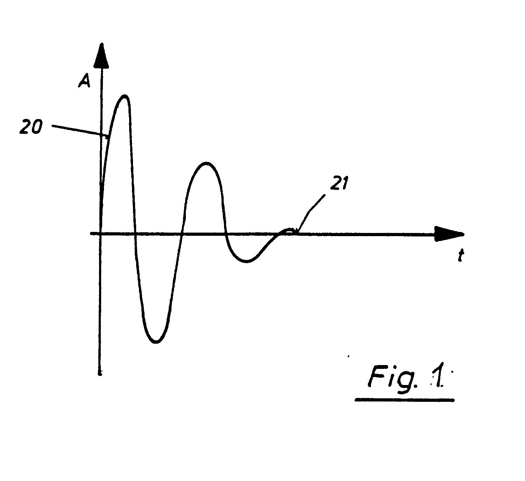

Fig. 1 zeigt schematisch ein Diagramm, wobei über der Zeit t die Amplitude eines erfindungsgemäßen Stoßimpulses aufgetragen ist. Es wird deutlich, daß dessen Anstiegsflanke 20 sehr steil ansteigt. Anschließend wird die Schwingung möglichst schnell auf den Wert Null gedämpft. Beim Biespiel erreicht sie diesen Dämpfungswert Null bei Pos. 21, d.h. nach zwei vollständigen Schwingungen.

**Patentansprüche**

1. Verfahren für den Einsatz in der medizinischen Diagnose, in der medizinische Rehabilitation und im Sportunterricht, zum Messen des Ortes mehrerer Meßpunkte (11) in einem vorgegebenen Koordinatensystem mit Hilfe von Ultraschallimpulsen, wobei man an jedem Meßpunkt (11) einen Ultraschallsender befestigt sowie wenigstens drei raumfeste Ultraschallempfänger vorsieht und man die Ultraschallsender nacheinander zu Stoßwellen erregt, deren jeweilige Laufzeit zu den Ultraschallempfängern man mißt und daraus mit Hilfe einer datenverarbeitenden Anlage den Ort der Meßpunkte bestimmt, wobei man durch ein oftmaliges Wiederholen der Messung in kurzen Abständen eine gegebenenfalls vorhandene Bewegung der Meßpunkte (11) mißt sowie gegebenenfalls daraus abgeleitete Größen, dadurch gekennzeichnet, daß die Ultraschallsender (11) in einer vorgegebenen Reihenfolge nacheinander zu Stoßimpulsen erregt werden, wobei diese Reihenfolge während der Messung gleich bleibt, und daß die zeitliche Verzögerung zwischen den Stoßimpulsen jeder Meßreihe als vorgegebene Verzögerungszeit so groß gewählt wird, daß, auch bei sich relativ zueinander bewegenden Ultraschallsendern (11), deren Ultraschallimpulse (14) stets in der gleichen Reihenfolge von den Ultraschallempfängern (12, 15) empfangen werden, wobei die vorgegebene Verzögerungszeit in einem Vorversuch bestimmt wird und wenigstens gleich der größten bei der Messung zu erwartenden Verzögerungzeit der bewegten Sender untereinander gewählt wird, und wobei die Anstiegsflanke (20) des Stoßimpulses möglichst steil ist und weniger als 90° beträgt und der Stoßimpuls höchstens sechs Schwingungen enthält.

2. Verfahren nach Anspruch 1, dadurch gekennzeichnet, daß bei Vorliegen von wenigstens vier der raumfesten Ultraschallempfänger (12, 15) wenigstens einer der Ultrachallempfänger (15) im Raum außerhalb derjenigen ersten Ebene angeordnet ist, die durch drei andere der Ultraschallempfänger (12) definiert ist.

3. Verfahren nach Anspruch 2, dadurch gekennzeichnet, daß der außerhalb dieser ersten Ebene angeordnete Ultraschallempfänger (15) zusammen mit zwei anderen derjenigen Ultraschallempfänger (12), die die erste Ebene definieren, eine weitere zweite Ebene definiert, die senkrecht zu der ersten Ebene angeordnet ist.

4. Abänderung des Verfahrens nach einem der Ansprüche 1—3, dadurch gekennzeichnet, daß die Ultraschallsender (1) und die Ultraschallempfänger (2) gegeneinander vertauscht sind.

5. Schaltungsanordnung zur Durchführung des Verfahrens nach einem der Ansprüche 1—4, wobei die Schaltungsanordnung an Meßpunkten befestigte Ultraschallsender (11), wenigstens drei raumfeste Ultraschallempfänger (12) und eine mit den Sendern (11) und Empfängern (12) verbundene datenverarbeitende Anlage aufweist, dadurch gekennzeichnet, daß die Schaltungsanordnung einen Zeitgeber (3) aufweist, der über einen Senderverstärker (4) den jeweiligen Ultraschallsender (1) mit einem Stoßimpuls beaufschlagt und der gleichzeitig einen Startimpuls an einen Zähler (5) abgibt, der von einem HF-Oszillator (6) mit Zählimpulsen beaufschlagt ist, daß der Ausgang des Zählers (5) mit einem empfängerseitigen Speicher (7) für die Zählimpulse verbunden ist, dessen Eingang über einen Empfängerverstärker (9) auch an den zugeordneten Ultraschallempfänger (2) angeschlossen ist und an dessen Ausgang die Anzeige (10) angeschlossen ist.

6. Ultraschallsender als Bestandteile der Schaltungsanordnung nach Anspruch 5 zur Durchführung des Verfahrens nach einem der Ansprüche 1—4, wobei die Schaltungsanordnung an Meßpunkten befestigte Ultraschallsender (11), wenigstens drei raumfeste Ultraschallempfänger (12) und eine mit den Sendern (11) und Empfängern (12) verbundene datenverarbeitende Anlage aufweist, dadurch gekennzeichnet, daß jeder Ultraschallsender (11) zwei gleichformatige, übereinander gelegte und miteinander verklebte Plättchen (12, 13) aus piezokeramischem Material aufweist, die so polarisiert sind, daß sie bei Anlegen einer Spannung vorgegebener Polarität an ihre metallisierten Außenflächen kugelschalenförmig in der einen Richtung und bei Spannungsumkehr in der anderen Richtung ausgelenkt werden und die an ihrer Unterseite im Bereich ihrer Kanten schwingungsgedämpft an einem Gehäuse (11a) befestigt sind.

7. Ultraschallsender nach Anspruch 6, dadurch gekennzeichnet, daß eine Lochblende (18) dicht vor der schallabstrahlenden Fläche der Plättchen (12, 13) angeordnet ist und daß auf die schallabstrahlende Fläche der Plättchen (12, 13) ein Schalltrichter (17) aufgesetzt ist.

**Revendications**

1. Procédé à utiliser dans le diagnostic médical, la réhabilitation médicale et l'enseignement sportif pour la mesure de l'emplacement de plusieurs points de mesure (11) dans un système de coordonnées prédéterminé à l'aide d'impulsions ultrasoniques, dans lequel on fixe un émetteur ultrasonique à chaque point de mesure (11) et on prévoit au moins trois récepteurs ultrasoniques fixes dans l'espace, et l'on excite l'un après l'autre les émetteurs ultrasoniques pour engendrer des ondes de choc, dont on mesure le temps de parcours respectif vers les récepteurs ultrasoniques et l'on détermine à partir de là, à l'aide d'une installation de traitement des données, l'emplacement des points de mesure, dans lequel on mesure, par une répétition fréquente de la mesure à de faibles intervalles, un mouvement éventuel des points de mesure (11) ainsi que des grandeurs qui en sont éventuellement dérivées, caractérisé en ce que, les émetteurs ultrasoniques (11) sont excités l'un après l'autre dans une séquence prédéterminée pour engendrer des ondes de choc, cette séquence restant constante pendant la mesure, et en ce que le retard de temps entre les ondes de choc de chaque série de mesure est choisi, en tant que temps de retard prédéterminé, suffisamment grand pour que, même pour des émetteurs ultrasoniques (11) se déplaçant les uns par rapport aux autres, leurs impulsions ultrasoniques (14) soient constamment reçues par les récepteurs ultrasoniques (12, 15) dans la même séquence, le temps de retard prédéterminé ètant déterminé dans un essai préalable et étant choisi au moins égal au plus grand des temps de retard à attendre, lors de la mesure, des émetteurs déplacés les uns par rapport aux autres, et le flanc montant (20) de l'impulsion de choc étant le plus raide possible et étant inférieur à 90°, et l'impulsion de choc contenant au plus six ondes.

2. Procédé selon la revendication 1, caractérisé en ce que, lors de la mise en place d'au moins quatre des récepteurs ultrasoniques (12, 15) fixes dans l'espace, au moins l'un des émetteurs ultrasoniques (15) est disposé, dans l'espace, en dehors du premier plan qui est défini par les trois autres récepteurs ultrasoniques (12).

3. Procédé selon la revendication 2, caractérisé en ce que, le récepteur ultrasonique (15) disposé en dehors de ce premier plan définit, avec deux autres de ces récepteurs ultrasoniques (12), qui définissent le premier plan, un deuxième plan, différent, qui est perpendiculaire au premier plan.

4. Modification du procédé selon l'une des revendications 1 à 3, caractérisée en ce que, les émetteurs ultrasoniques (1) et le récepteur ultrasonique (2) sont échangés les uns avec les autres.

5. Agencement de circuit pour la mise en oeuvre du procédé selon l'une des revendications 1 à 4, l'agencement de circuit comportant des émetteurs ultrasoniques (11) fixés en des points de mesure, au moins trois récepteurs ultrasoniques (12) fixes dans l'espace et une installation de traitement de données reliée aux émetteurs (11) et aux récepteurs (12), caractérisé en ce que, l'agencement de circuit présente une horloge (3), qui applique une impulsion de choc, par un amplificateur d'émetteur (4), aux émetteurs ultrasoniques respectifs (1), et qui simultanément envoie une impulsion de départ à un compteur (5) auquel sont appliquées, par un oscillateur HF (6) des impulsions de comptage, en ce que la sortie du compteur (5) est reliée à une mémoire (7) d'impulsions de comptage du côté récepteur, dont l'entrée est également reliée, par un amplificateur de récepteur (9) au récepteur ultrasonique associé (2), et à la sortie duquel est raccordé l'affichage (10).

6. Emetteurs ultrasoniques agissant en tant que constituants de l'agencement de circuit selon la recendication 5, destinés à la mise en oeuvre du procédé selon l'une des revendications 1 à 4, l'agencement de circuit comprenant des émetteurs ultrasoniques (11) fixés aux points de mesure, au moins trois récepteurs ultrasoniques (12) fixes dans l'espace, et une installation de traitement de données reliée aux émetteurs (11) et aux récepteurs (12), caractérisé en ce que, chaque émetteur ultrasonique (11) comporte deux petites plaquettes (12, 13) en matière piézocéramique, de même format, superposées l'une à l'autre et collées l'une à l'autre, qui sont polarisées de telle façon qu'elles soient déformées en forme de coquilles sphériques dans un sens, lors de l'application d'une tension de polarité prédéterminée à leurs surfaces extérieures métallisées, et dans l'autre sens lors de l'inversion de tension, et qui sont fixées à leurs côtés inférieurs dans la zone de leurs arêtes à un boîtier (11a), leurs oscillations étant amorties.

7. Emetteur ultrasonique selon la revendication 6, caractérisé en ce que, un écran à trou (18) est disposé de façon étanche devant la surface émettrice d'ultrasons des plaquettes (12, 13) et en ce qu'un diffuseur sonique (17) est placé sur la surface émettrice de son des plaquettes (12, 13).

**Claims**

1. Method for application in the medical diagnosis, in the medical rehabilitation and in athletics lessons for measuring the location of several measuring points (11) in a predetermined coordinate system by means of ultrasonic impulses wherein at each measuring point (11) an ultrasonic transmitter is fastened and at least three stationary ultrasonic recorders are provided, and the ultrasonic transmitters are energized to impulse waves one after another measuring the running time of the impulses to each to the ultrasonic recorders, thereby determinating by means of a computer the location of the measuring points, and that by often repetition of the measurement within short intervals measuring a possible movement of the measuring points (11) as well as possibly values traced therefrom, characterized in that the ultrasonic transmitters (11) are energized in a predetermined series to impact impulses wherein this series is maintained during the measurement, and that the temporary delay between the impact impulses of each measuring series as predetermined delay time is selected in such dimension that also with the ultrasonic transmitters (11) moving relatively to each other, the ultrasonic impulses (14) of which are always received in the same series by the ultrasonic recorders (12, 15), wherein the predetermined delay time is determined in a preliminary test and is at least like the greatest delay time to be expected in the measurement of the moving transmitters between each other is selected, and the wave flank (20) of the impact impulse is possibly steep and is less than 90°, and the impact impulse is containing at the most six oscillations.

2. Method according to claim 1, characterized in that in the presence of at least four of the stationary ultrasonic recorders (12, 15) at least one of the ultrasonic recorders (15) is provided in the space outside of that first plane which is defined by three other ultrasonic recorders (12).

3. Method according to claim 2, characterized in that the ultrasonic recorder (15) provided outside this first plane together with two other of those ultrasonic recorders (12) which are defining the first plane, is defining a second plane which is provided vertically to the first plane.

4. Modification of the method according to one of claims 1 to 3, characterized in that the ultrasonic transmitters (1) and the ultrasonic recorders (2) are exchanged with each other.

5. Switching arrangement for the execution of the method according to one of claims 1 to 4, wherein the switching arrangement is provided with ultrasonic transmitters (11) fastened at measuring points, at least three stationary ultrasonic recorders (12) and a computer connected to the transmitters (11) and the recorders (12), characterized in that the switching arrangement is provided with a time-signal transmitter (3) which over a transmitter amplifier (4) is acting upon each ultrasonic transmitter (1) with an impact impulse and which at the same time is discharging a starting impulse to a counter (5) which is acted upon by a HF-oscillator (6) with counting impulses, that the exit of the counter (5) is connected with an entry-memory (7) for the counting impulses the input of which over a recorder amplifier (9) also is connected with the collated ultrasonic recorder (2) and to the exit of which the reading (10) is connected.

6. Ultrasonic transmitters as components of the switching arrangement according to claim 5 for the execution of the method according to claims 1 to 4, wherein the switching arrangement is provided with ultrasonic transmitters (11) fastened at the measuring points, at least three stationary ultrasonic recorders (12) and a computer connected to the transmitters (11) and the recorders (12), characterized in that each ultrasonic transmitter (11) is provided with two uniform superposed small plates (12, 13) glued to each other and made of piezoceramic material which are polarized in such way that when applying a voltage of predetermined polarity to its metallized outer planes same will be deflected spherically in one dierction and when reversing the voltage in the other direction, and which are fastened at its lower side in the area of its edges oscillation damped at a housing (11a).

7. Ultrasonic transmitter according to claim 6, characterized in that a diaphragm (18) with an aperture is provided closely in front of the sound emitting plane of the small plates (12, 13), and that onto the sound emitting plane of the small plates (12, 13) a sound funnel (17) is set up.

Fig. 1

*Fig.2*

Fig.3

Fig. 4

Fig. 5

Fig. 6

Fig. 7